# EUROPEAN PATENT SPECIFICATION

(11) **EP 1 636 587 B1**
(45) Date of publication and mention of the grant of the patent: **28.04.2010**
(21) Application number: 04734768.7
(22) Date of filing: 25.05.2004
(51) Int. Cl.: G01N 33/68, C12P 21/02, C12N 5/10, C12Q 1/68, A61K 31/404

(54) **DIAGNOSING LIVER CIRRHOSIS USING AN ANTIBODY SPECIFIC FOR HUMAN PROTOONCOGENIC PROTEIN**
DIAGNOSE VON LEBERZIRRHOSE MITTELS EINES FÜR MENSCHLICHES PROTOONKOGENES PROTEIN SPEZIFISCHEN ANTIKÖRPERS
DIAGNOSTIC DE LA CIRRHOSE DU FOIE UTILSANT UN ANTICORPS SPECIFIQUE D'UNE PROTEINE PROTO-ONCOGENE HUMAINE

(30) Priority: 26.05.2003 KR 2003033308
(43) Date of publication of application: 22.03.2006
(73) Proprietor: Kim, Jin-Woo, Seoul 135-976 (KR)
(72) Inventor: Kim, Jin-Woo, Seoul 135-976 (KR)
(74) Representative: Habermann, Gert
(86) International application number: PCT/KR2004/001242
(87) International publication number: WO 2004/104598

(56) References cited:
- WO-A-02/44370
- KR-A- 2002 041 550
- US-B1- 6 265 161
- YOON SEUNG-KEW ET AL: "Development of a new tumor marker against human hepatocellular carcinoma" HEPATOLOGY, vol. 36, no. 4 Part 2, October 2002 (2002-10), page 302A, XP009066894 & 53RD ANNUAL MEETING ON THE LIVER; BOSTON, MA, USA; NOVEMBER 01-05, 2002 ISSN: 0270-9139
- LACOVAZZI P.A. ET AL.: 'Serum 90K/MAC-2PB Glycoprotein in Patients with Liver Cirrhosis and Hepatocellular Carcino: a Comparison with alpha-Fetoprotein' CLIN. CHEM. LAB. MED. vol. 39, no. 10, October 2001, pages 961 - 965, XP003000888
- RAEDELE J. ET AL.: 'Alpha-Fetoprotein and p53 Autoantibodies in Patients with Chronic Hepatitus C' DIG. DIS. SCI. vol. 40, no. 12, December 1995, pages 2587 - 2594, XP003000847
- KO J. ET AL. ONCOGENE vol. 22, no. 30, 24 July 2003, KOREA, pages 4679 - 4689, XP002332525
- YOON S.K. ET AL.: 'The human cervical cancer oncogene protein is a biomarker for human hepatocellular carcinoma' CANCER RES. vol. 64, no. 15, 01 August 2004, KOREA, pages 5434 - 5441, XP002332526

## Description

### Technical Field

The present invention relates to a method for diagnosing liver cirrhosis in vitro using an antibody specifically binding to a protein expressed from human proto-oncogene HCCR-2.

### Background Art

Liver cirrhosis is referred to as liver fibrosis in a broad sense in that it causes tubercles in liver of a patient. As an end-stage chronic liver disease, the liver cirrhosis causes persistent and recurring diffuse liver injuries, leading to fibrosis and formation of tubercles in liver cells (Ikeda et al., Hepatology 18:47-53, 1993; Minami H and Okanoue T, Int Med. 80: 646-649, 1997; Kiyosawa K, Hepatology Research 24: S40-S45, 2002). Major factors for inflammation of liver Include virus, medicinal constituents, alcohol, metabolic diseases, chronic cholestasis, hepatic vein bloods flow occlusion. Liver cirrhosis accounts for 60-90% of liver cancer cases, and liver cancer arises in 5-20% of liver cirrhosis patients, suggesting that liver cirrhosis increases the risk of developing liver cancer. Hence, there is a pressing need for early diagnosis of liver cirrhosis and elimination of major factors causing liver cirrhosis (Velazquez et al., Hepatology 37(3): 520-527, 2003). In addition to the viral factor, risk factors associated with liver cirrhosis include alcohol abuse, chemical substances, hemochromatosis and so on. Therefore, it is quite important to perform early detection, elimination, Inoculation and quarantine of such diseases and risk factors thereof.

Liver cirrhosis presented with clinically normal features without complications is referred to as compensated liver cirrhosis. Liver cirrhosis concomitant with a variety of complications is referred to as decompensated liver cirrhosis.

Abdominal laparoscopic examination and liver organ examination has gained popularity as the foremost tests in diagnosing liver cirrhosis. To directly observe the internal structure of liver, the abdominal laparoscopic technique is performed in the inferior of the abdomen through a small incision, that is, an endoscope is inserted through a small entrance incision in the abdominal cavity. When a hard and lumpy surface is visualized through the abdominal laparoscopic examination, the liver organ is diagnosed as being affected by cirrhosis. Also, when liver fibrosis is visualized through the liver organ examination, the presence or severity of liver cirrhosis is diagnosed. However, such examination can be a psychological burden to a patient. Also, an accurate diagnosis for liver cirrhosis is often based on a combination of a variety of methods or techniques, including doctor's medical advice, blood test, ultrasonography, computerized tomography (CT).

In a case of liver cirrhosis, the level of serum alanine aminotransferase (ALT) (equivalent to conventional GOT or GPT level) is not so high and is substantially normal or less than two times the normal level, which makes it impossible to be used as effective diagnostic indicator for liver Inflammation. In particular, even in cases involving compensated liver cirrhosis, there are considerable liver cells that normally function. Thus, the level of albumin or bilirubin falls under a substantially normal range. On the other hand, in cases involving decompensated liver cirrhosis, the level or albumin may decrease or the level of bilirubin may increase. The presence of normally functioning liver cells is quite Important for liver cirrhosis or advanced chronic liver disease patients, and albumin or bilirubin is only a rough indicator for the presence of normal liver cells. If normal liver cells, which produce blood coagulating factors, are insufficient, coagulation of blood may be delayed. A prothrombin time (PT) examination is performed to directly evaluate a complete coagulation of blood, which also acts as an indicator to evaluate remaining liver function. Liver cirrhosis results in an enlargement of the spleen, in which a large quantity of platelets is entrapped. Thus, a reduction in the platelet level due to unknown cause is suspected of having liver cirrhosis.

Serological marker examination against hepatitis virus like in chronic hepatitis also Importantly acts as an indicator for liver cirrhosis. In Korea, approximately 60% of liver cirrhosis is caused by hepatitis B virus (HBV) and approximately 20% of liver cirrhosis is caused by hepatitis C virus (HCV). Thus, it can be said that a positive HBV or HCV marker suggests a high risk factor for chronic liver diseases. In addition, when a patient is presented with other lesions suggesting liver cirrhosis, it is natural to diagnose the patient's lesion as liver cirrhosis.

A non-specific Increase in the serum alpha-fetoprotein (APF) level, which is an assay for early detection of liver cancer, may be observed In some patients having chronic hepatitis or cirrhosis and not having HCC lesions (Adinolfi A et al., J Med Genet. 12(2_" 138-151, 1975; Lock AS and Lai CL Hapatology 9(1). The level of alpha-fetoprotein (AFP) has been advantageously used in diagnosing liver cirrhosis as one of screening factors for early detection of liver cirrhosis based on the finding that the concentration of AFP in normal adult sera is almost zero but drastically increases in many patients with liver cirrhosis. In fact, however, the concentration of AFP may increase in chronic hepatitis or liver cirrhosis. For these reasons, although AFP testing is regarded as an essential step in screening patients with HCCs among patients with chronic liver diseases to some extents, diagnosis of liver cirrhosis based on the AFP level requires a number of considerations, which means that there is a need for research into new useful diagnostic methods. Thus, as part of such research, in order to achieve early diagnosis of and effective prediction of liver cirrhosis, development of a new serological test with Improved sensitivity and specificity would be highly desirable.

Accordingly, in order to develop a method for effective early diagnosis of liver cirrhosis, the present inventors undertook earnest research and studies and Identified that liver cirrhosis could be effectively detected by an antigen-antibody binding reaction using antibodies specifically binding to a protein expressed from human proto-oncogene HCCR-2 deposited at the GenBank as Accession No. AF315598, as described In Korean Patent Publication No. 2002-41550 and WO 02/44370. This finding has led the present inventors to complete the present invention.

### Disclosure of the invention

To solve the above problems, it is an object of the present invention to provide a method for effectively detecting liver cirrhosis in an early stage using human proto-oncogene HCCR-2 in serum.

To accomplish the above object of the present invention, there is provided the use of a specific antibody against HCCR-2 protein as defined in claim 1.

### Brief Description of the Drawings

FIG. 1 illustrates results of immuno-blotting performed on HCCR-2 recombinant protein isolated and purified from Escherichia coli (E. coli) BL21 strain transformed into pMAL-p2X/HCCR-2 vector; and
FIG. 2 Illustrates diagnosis results of detecting the expression of HCCR-2 protein in liver cirrhotic serum and normal serum by a diagnostic kit using HCCR-2 polyclonal antibody.

### Best mode for carrying out the Invention

The human proto-oncogene HCCR-2 (GenBank Accession No. AF315598; Korean Patent Publication No. 2002-41550), which is positioned in the long arm of Chromosome No. 12 (12q) and has an open reading frame, acts as a carcinogenic gene when over-expressed in mammals, and a protein having a size of about 36 kDa (to be referred to as HCCR-2 protein) is derived therefrom.

The HCCR-2 protein specific antibody is preferably a polyclonal antibody. More preferably, the HCCR-2 protein specific antibody is a polyclonal antibody purified from the serum obtained by immunizing HCCR-2 antigen protein in a rabbit.

To synthesize an antibody specifically recognizing HCCR-2 protein, HCCR-2 protein is first acquired. HCCR-2 protein can be synthesized using known amino acid sequences or produced in recombinant protein types by genetic engineering methods. For example, HCCR-2 recombinant protein can be prepared by a method comprising preparing an expression vector of expressing HCCR-2 protein in the form of a recombinant protein using the base sequence of the HCCR-2 gene set forth in the NIH program GenBank database; obtaining a transformant by transforming the expression vector into E.coli to produce HCCR-2 recombinant protein; and cultivating the transformant to isolate/purify the human proto-oncogene HCCR-2 recombinant protein.

A recombinant protein having maltose fused to N-terminal is produced from E. coli BL21 transformed into pMAL-p2X/HCCR-2 vector containing amino acid sequences 112-304 of a HCCR-2 gene, and then treated with an appropriate enzyme to isolate/purify HCCR-2112-304 protein from which maltose is removed to then be used as antigen protein. In order to identify that the thus produced protein is HCCR-2112-304 recombinant protein, specific detection of HCCR-2 recombinant protein having a molecular weight of about 66 kDa (45 kDa of pMAL plus 16 kDa of HCC-2112-304) is observed by Western blot analysis.

The immunized rabbits necessary for development of polyclonal antibodies are generated by well mixing the HCCR-2 recombinant protein and an equivalent amount of Freund's complete adjuvant until the mixture is emulsified and Injected into rabbits intraperitoneally, followed by booster injection for the purpose of increasing the immunogenicity of the rabbits. Preferably, only the HCCR-2 recombinant protein is further administered three times into the rabbits via an intraperitoneal route, rather than through the use of the adjuvant.

The rabbit sera immunized through injection of the HCCR-2 recombinant protein antigen are collected and antibody titers are measured.

Diagnosing cancer by a antigen-antibody binding reaction using the thus produced HCCR-2 specific antibody protein can be made by determining the expression of this protein in a sample in vivo, taken from a sample. The level of expression can be detected by technique known in the art, including enzyme-linked immunosorbent assay (ELISA), radioimmunoassay (RIA), sandwich assay, and Western blotting or immunoblotting analysis on a polyacrylamide gel.

As the sample in vivo (specimen), tissues, sera or platelets, most preferably sera, are preferably used.

In a preferred embodiment, the ELISA technique using HCCR-2 protein specific antibodies is carried out through the following steps:
1) placing an HCCR-2 specific antibody into a reactor coated with a sample and a control group to induce an antigen-antibody reaction;
2) detecting an antigen-antibody reaction product using a secondary antibody-label conjugate and a color-developing substrate solution of a labeling substance; and
3) comparing the detection result of the sample with that of the control group.

A large amount of the sample can be analyzed using known technique such as ELISA, biological microchip or automated microarray system. The biological micro chip can detect an antigen for the HCCR-2 specific antibody protein by fixing the HCCR-2 specific antibody protein on a biological microchip, causing a reaction between the same and a sample in vivo collected from an individual.

The present invention provides the use of an antibody specifically reacting with the HCCR-2 for early diagnosis of liver cirrhosis in vitro.

Preferably, the diagnosis of liver cirrhosis according to the present invention makes use of a diagnostic kit comprising:
1) a specific antibody against HCCR-2;
2) a positive control group containing a HCCR-2 standard antigen and a negative control group containing antiserum of an animal into which the antigen is not injected.
More preferably, the kit further comprises:
3) a secondary antibody conjugate having a conjugated labeling substance producing a color by reacting with a substrate;
4) a color-developing substrate solution reacting with the labeling substance to produce a color;
5) a washing solution to be used in each step; and
6) an enzymatic reaction stopping solution.

The diagnostic kit can diagnose liver cirrhosis by quantitatively or qualitatively analyzing an antigen of the antibody protein by the antigen-antibody binding reaction. The antigen-antibody binding reaction can be detected by technique generally known in the art, including ELISA, RIA, sandwich assay, Western blotting on a polyacrylamide gel, and immunoblotting analysis. For example, the diagnostic kit can be provided to be used for ELISA using a 96-well microtiter plate coated with recombinant monoclonal antibody protein.

Examples of the reactor useful to coat the antibody protein thereon include a nitrocellulose membrane, a 96-well plate made of a polyvinyl resin, a 96-well plate made of a polystyrene resin, and slide glass.

As described above, the antibody used according to the present invention is preferably a polyclonal antibody. The HCCR-2 protein specific antibody is preferably coated at a rate of about 1 to 10 µg/100 µl for each reactor.

The control groups contained in the diagnostic kit used according to the present invention include positive control groups and negative control groups. The positive control groups are mixtures containing HCCR-2 protein standard antigen, and the negative control groups are animal sera uninfected with HCCR-2 protein antigen. In Examples, HCCR-2 protein standard antigen solutions having various protein concentrations of 0 ng/mℓ (A), 20 ng/ml (B), 40 ng/mℓ (C), 80 ng/mℓ (D), 160 ng/mℓ (E), 320 ng/mℓ (F) and 640 ng/mℓ (G), were used.

As the secondary antibody labeling substance, known labeling substances inducing color development are preferably used, and examples the labeling substance useful in the present invention include horseradish peroxidase (HRP), alkaline phosphatase, colloid gold, fluorescein such as poly L-lysine-fluorescein isothiocyanate (FITC) or rhodamine-B-isothiocyanate (RITC), and a dye. Goat anti-rabbit IgG-HRP conjugate (IgG-HRP conjugate), for example, is used.

Preferably, the chromogen used varies according to the labeling substance involving the color development, and usable examples thereof include 3,3',5,5'-tetramethyl bezidine (TMB), 2,2'-azino-bis(3-ethylbenzothiazoline-6-sultonic acid) (ABTS), and o-phenylenediamine (OPD). More preferably, the chromogen is provided in a state in which it is dissolved in a buffer solution (0.1 M NaAc, pH 5.5). The chromogen such as TMB is decomposed by HRP used as the labeling substance of the secondary antibody conjugate to produce a color-developing precipitate. The level of precipitation of the color-developing precipitate is observed by naked eye, thereby determining the presence of HCCR-2 protein antigen.

The washing solution preferably include a phosphate buffer solution, NaCl, and Tween 20, more preferably a buffer solution containing 0.02 M phosphate buffer solution, 0.13 M NaCl, and 0.05% Tween 20. Following antigen-antibody binding reaction, an appropriate amount of the washing solution is supplied Into the reactor having undergone the reaction between the secondary antibody and the antigen-antibody conjugate. Washing with the washing solution is repeatedly performed 3 to 6 times. 0.1% BSA containing phosphate buffer is preferably used as the blocking solution and 2 N sulfuric acid solution Is preferably used as the enzymatic reaction stopping solution.

The method of diagnosing liver cirrhosis in an early stage by detecting HCCR-2 antigen in a specimen sample using the diagnostic kit will now be described. HCCR-2 polyclonal antibodies and specimen samples in the positive and negative control groups are reacted, respectively, and washed with the washing solution. Then, the secondary antibody conjugate labeled with a labeling substance producing a color by the reaction with the substrate is added to the resultant product and washed again with the washing solution. Then, the substrate containing solution is added to the resultant production to Induce color development. Then, the absorbance at 450 nm is measured. Here, the average absorbance of the standard antigen solution A should be greater than or equal to 0.000 and less than or equal to 0.200. The average absorbance of the standard antigen solution F should be greater than or equal to 1.200 and less than or equal to 3.000. The mean value between the absorbance values of the standard antigen solutions A and F is set as a cut-off value to then be used to determine samples as positive or negative samples. When the absorbance of a sample Is greater than that of the standard antigen solution F, the sample is diluted and the absorbance thereof is then measured again. The sample having absorbance above the cut-off value is identified as being positive, and the sample having absorbance below the cut-off value is identified as being negative.

It was confirmed that the diagnosis of liver cirrhosis according to claim 1 using the diagnostic kit containing the human proto-oncogene HCCR-2 specific antibody had higher accuracy and reproducibility. Conventionally, an AFP test tool used for diagnosis of liver cancer has also been used as a serological diagnostic kit for liver cirrhosis, which is, however, very poor in accuracy. However, the use of serological HCCR-2 test for early diagnosis of liver cirrhosis according to claim 1 showed a diagnosing accuracy of about 95.1%, which is statistically significantly higher than the conventional AFP test.

The present invention will now be illustrated in detail by the following non-limiting.

### <Example 1> Production of HCCR-2 Polyclonal Antibody

### <1-1> Production of HCCR-2 Recombinant Protein

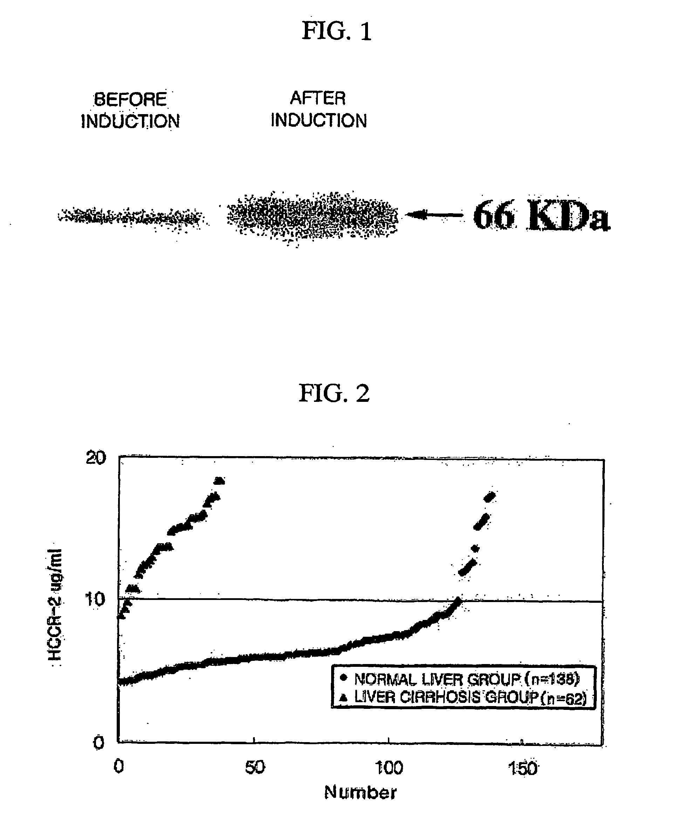

In order to produce human proto-oncogene HCCR-2 specific antibody as a labeling substance for diagnosis of liver cirrhosis, pET-32b(+)/HCCR-2 vector was prepared by inserting part of human proto-oncogene HCCR-2 corresponding to amino acid sequence ID NOS: 112-304 of GenBank Accession No. AF315598 into multicloning sites of pET-32(+) vector (New England biolabs, MA). The vector was transformed into E. coli BL21 (DE3) (Novagen, WI) and treated with 1 mM isopropyl β-D-thiogalacto-pyranoside (IPTG) (manufactured by Sigma Chemical Co.) to induce expression, producing 66 KDa HCCR-2112-304 fused protein having 45 KDa maltose protein fused thereto and purifying the same with an amylase resin kit (New England biolabs, MA) (International Patent Application Publication No. WO 02/44370 A1). The purified recombinant protein was subjected to immonoblotting, confirming that a large amount of about 66 kDa fusion protein was included (FIG. 1). The thus isolated and purified HCCR-2 recombinant protein was used as an antigen in the antigen-antibody binding reaction for screening and analyzing fused cell lines producing polyclonal antibodies for immunization of rabbits.

### <1-2> Immunization of Rabbits

In order to yield immunized rabbits necessary for production of antibodies specific to the HCCR-2 recombinant protein prepared In Example <1-1>, New Zealand White (NZW) rabbits (average weight of about 2.5 kg) was used. 200 µg/ℓ of the HCCR-2 recombinant protein and 200 µg/ℓ of Freund's complete adjuvant (manufactured by Sigma Chemical Co.) were mixed and emulsified. 2 mℓ of the resultant emulsion was subcutaneously given at 8 dorsal sites at a rate of 0.25 mt per rabbit. After the first injection, booster injection was performed several times for 2 weeks at two-week intervals in the same manner as in the first injection by emulsifying the immunized antigen using Freund's incomplete adjuvant.

### <1-3> Isolation of Rabbit Serum and Screening of Specific Polyclonal Antibody

At a 2-week Interval after day 5 from the last inoculation, blood was collected from the artery of the rabbits immunized in Example <1-2> and serum was isolated therefrom to be stored at -20°C until it is used in various experiments. Antibody specificity of the serum was examined, and the result showed that it specifically reacted with only human proto-oncogene HCCR-2 recombinant protein. In order to screen sera specifically reacting with the HCCR-2 protein antigen among the sera isolated and purified from the E. coli transformant prepared in Example <1-1> was subjected to ELISA.

In detail, in order to suppress non-specific immune responses, 96-well plate (Falcon Co., USA) was coated by applying 1% skim milk-PBS thereto and allowing the same to stand at room temperature for 1 hour, and the recombinant proteins were coated onto each well in an amount of 1 µg/well. The rabbit serum fluid was diluted with a 2% BSA containing PBS solution in various concentrations of 1 × 10³, 1 × 10⁴, 1 × 10⁵, 1 × 10⁸, and 1 × 10⁷, and the resulting diluted solution was added to each well in an amount of 100 µl per well. Thereafter, an antigen-antibody binding reaction was induced to take place at 37°C for 2 hours, followed by washing three times with PBS solution. Then, each 100 µl of goat anti-rabbit IgG (manufactured by Sigma Co., USA) as a secondary antibody, diluted to 1/10,000 with a PBS buffer solution containing 2% (W/V) BSA was added to each well and reacted at 37°C for 1 hour.

Thereafter, 100µl of a substrate solution obtained by mixing 10 ml of 0.1 M phosphate buffered citrate (pH 5.0), 1 mg of 3.3', 5.5'-tetramethylbenzidine (TMB) (manufactured by Sigma Co., USA) and 20 µl of 35% hydrogen peroxide, was added to each well to induce an enzymatic reaction. The enzymatic reaction was maintained at room temperature for 15 minutes and the same amount of 2 N sulfuric acid solution was then added thereto to stop the enzymatic reaction. The extent of color development was observed at 450 nm. From polyclonal antibodies having HCCR-2 protein specific antibodies, serum with antibody titer of 10 times greater than that of the negative control group, as measured by ELISA, were further screened, and characteristics of the screened cells were analyzed.

### <Example 2> ELISA using HCCR-2 Polyclonal Antibody

The liver cirrhosis was diagnosed by ELISA using HCCR-2 polyclonal antibody in the following manner. First, wells were coated with the sample; Second an ELISA plate wells were coated with HCCR-2 specific polyclonal antibodies Isolated and purified from the rabbit. Third, step detecting the presence of HCCR-2 antigen-antibody binding in the sample.

### <2-1> Coating with sample on the wells

Liver cirrhosis and hepatitis sera and normal liver serum were used as specimen samples. To obtain a standard curve for measurement of cut-off values, standard antigen solutions A, B, C, D, E, F and G were prepared by diluting HCCR-2 recombinant protein at various concentrations of 0 ng/m/mℓ, 20 ng/ml, 40 ng/ml, 80 ng/ml, 160 ng/ml, 320 ng/ml and 640 ng/ ml, respectively.

Each 100µl of the respective specimen samples was distributed to each 96-well ELISA flat-bottomed plate and reacted at 37°C for 4 hours, followed by washing 4 times with a washing buffer solution(PBS including 0.05% Tween 20, Ph 7.4). Here, the standard antigen solutions prepared above were used as positive control groups and normal rabbits sera were used as negative control groups.

### <2-2> Addition of HCCR-2 Polyclonal Antibody

HCCR-2 protein specific polyclonal antibodies according to example <1-3> were placed in each well coated with the specimen samples, covered with a lid and allowed to stand at 4°C for 16 to 18 hours. The polyclonal antibodies were diluted in 0.5 M carbonate buffer (pH 9.6) in a concentration of 5 µg/ml and 100 µl of the diluted solution was added to each well. As a control group, the normal rabbit serum that is not infected with HCCR-2 protein was 500-fold diluted in a carbonate buffer solution and distributed to each well (100 µl/well).

Then, the wells of the plate were washed 4 times with a washing buffer solution. In order to block non-specific protein binding sites, a blocking solution (PBS buffer solution (pH 7.4) containing a 2% BSA) was distributed to each well (300 µl/well) and allowed to stand at 37°C at 2 hours.

### <2-3> Detection of Antigen-Antibody Complex

100 µl of a 10,000-fold dilution of horsedarish peroxidase conjugated goat anti-rabbit IgG secondary antibody was added to each well, and the plate was allowed to stand at 37°C for 1 hour, followed by washing 4 times with a washing buffer solution. Subsequently, 1 mg of 3.3',5.5'-tetramethylbenzidine (TMB) (Sigma Co., USA) as a substrate was dissolved in 10 ml of a citrate buffer solution (pH 5.0) and 2 µl of 35% hydrogen peroxide was added thereto, thereby preparing a substrate solution. 100 µl of the prepared substrate solution was distributed to each well and reacted at room temperature for 15 minutes without exposure to light. Thereafter, 50 µl of 2 N H₂SO₄ solution was added to stop the reaction and the absorbance at 450 nm was measured.
For each specimen sample, the absorbance by HCCR-2 antigen was inferred as the remainder obtained by subtracting the absorbance of wells coated with only HCCR-2 fusion protein as the positive control group and PBS as the negative control group from the absorbance of the sample.
In the same manner, after the absorbance values of the standard solutions were calculated the mean value between the absorbance values of the standard antigen solutions A and F was set as a cut-off value. The sample having absorbance above the cut-off value is identified as being positive, and the sample having absorbance below the cut-off value Is Identified as being negative.

Here, the average absorbance of the standard antigen solution A should be greater than or equal to 0.000 and less than or equal to 0.200. The average absorbance of the standard antigen solution F should be greater than or equal to 1.200 and less than or equal to 3.000.

The cut-off value was determined as 10 µg/ml from the standard curve prepared using the HCCR-2 standard antigen solutions. Based on the cut-off value, the absorbance values of the respective samples were compared to determine whether they are positive or negative. The comparison results of concentrations of HCCR-2 protein in blood collected from the liver cirrhosis group and normal group are shown in FIG. 2.

### <Example 3> Confirmation of Liver Cirrhosis Diagnostic Efficiency by ELISA using HCCR-2 Polyclonal Antibody

### <3-1> Diagnosing accuracy of AFP and HCCR-2 Kits for Liver Cirrhosis Patient Group

In order to confirm the liver cirrhosis diagnostic efficiency using the HCCR-2 polyclonal antibody, measurement by ELISA using the HCCR-2 specific antibody described in Example 2 and measurement by the conventional alpha-fetoprotein (AFP) test, which has conventionally been used for diagnosis of HCC or liver cirrhosis, were compared. AFP levels were measured using ELSA2-AFP kit commercially available from CIS Bio International, France.

To determine whether samples are positive or negative based on diagnosing results, the cut-off value for AFP was set to 20 ng/ml and the cut-off value for HCCR-2 was set to 10 µg/ml, which was obtained from the standard curve. A difference in the reactivity between AFP positives and HCCR-2 positives within each of the liver cirrhosis and normal maternal groups was compared with data measured by McNemar test. From liver cirrhosis and normal groups, HCCR-2 sensitivity, specificity, pseudo-positive/negative rate and 95% confidence Intervals were inferred.

A difference in the HCCR-2 positive reactivity between each of the respective groups was compared with data measured by Fisher's exact test. A difference between AFP positive reactivity and HCCR-2 positive reactivity of each group was compared with data measured by McNemar test. SAS variance 6.12 (SAS/STAT Software: Changes and Enhancements through Release 6.12. Cary, NC: SAS Institute, 1997) was used in all statistical analyses with a significance level of 0.05.

Table 1 shows comparison results of diagnosing accuracy of HCCR-2 and AFP kits for 61 out of 62 liver cirrhosis patients, exclusive of a single patient in HCC group consisting of 62 liver cirrhosis patients. The results showed that the diagnosing accuracy based on HCCR-2 was 95.1%, which is significantly higher than that based on AFP, 18.0% (chi_M^2=43.614, df=1, P=0.0001, McNemar test).

**Table 1**

| | | AFP | | |
|---|---|---|---|---|
| | | Positive | Negative | Total |
| HCCR-2 | Positive | 9 | 42 | 58 (95.1%) |
| | Negative | 2 | 2 | 3 |
| | Total | 11 (18.0%) | 50 | 61 (100%) |

### <3-2> Confirmation of Utility of HCCR-2 Kit as Diagnostic Kit for Liver cirrhosis

As shown in Tables 2 and 3, 62 liver cirrhosis patients and 138 normal persons were diagnosed with ELISA using the HCCR-2 specific antibody described in Example 2. Sensitivity of the HCCR-2 kit was 95.2% and specificity thereof was 91.3%, pseudo-positives and pseudo-negatives were 16.9% and 2.3%, respectively. Also, the overall diagnosing accuracy was 92.5.

**Table 2**

| | | HCCR-2 | | |
|---|---|---|---|---|
| | | Positive | Negative | Total |
| Group | Uver cirrhosis | 59 | 3 | 62 |
| | Normal | 12 | 126 | 138 |
| | Total | 71 | 129 | 200 |

**Table 3**

| Measure | Value (%) | 95% Confidence Interval |
|---|---|---|
| Sensitivity | 95.2 | 89.8 ∼ 100 |
| Specificity | 91.3 | 86.6 ∼ 96.0 |
| Pseudo-positivity | 16.9 | 8.1 ∼ 25.6 |
| Pseudo-negativity | 2.3 | 0 ∼ 4.9 |
| Accuracy | 92.5 | 88.9 ∼ 96.2 |

As described above, the diagnosis of liver cirrhosis according to claim 1 using the kit containing the human proto-oncogene HCCR-2 specific antibody has higher accuracy and reproducibility than the conventional AFP test tool.

## Claims

1. Use of a specific antibody against human proto-oncogene HCCR-2 protein (GenBank Accession No. AF315598) for diagnosing liver cirrhosis *in vitro.*

2. The use of claim 1 wherein the diagnosing is performed by measuring the level of expression HCCR-2 protein in a specimen sample by an antigen-antibody binding reaction.

3. The use of claim 2 wherein the antigen-antibody binding reaction is detected by any one technique selected from the group consisting of enzyme-linked immunosorbent assay (ELISA), radioimmunoassay (RIA), sandwich assay, and Western blotting, immunoblotting analysis or immunohistochemistry on a polyacrylamide gel.

4. The use of claim 2 or 3 wherein the HCCR-2 specific antibody is a polyclonal antibody.

5. The use according to any one of claims 2 to 4 wherein the specimen sample is a tissue, serum or blood plasma.

6. The use of claim 2 wherein the specific antibody against human proto-oncogene HCCR-2 protein is part of a diagnostic kit further comprising a positive control group containing a HCCR-2 standard antigen and a negative control group containing antiserum of an animal into which the antigen is not injected.

7. The use of claim 6 wherein the kit further comprises
a) secondary antibody conjugate having a conjugated labelling substance producing a color by reacting with a substrate;
b) a color-developing substrate solution reacting with the labelling substance to produce a color;
c) a washing solution to be used in each step; and
d) an enzymatic stopping solution.

8. The use of claim 7 wherein the labelling substance of the secondary antibody conjugate is selected from the group consisting of horseradish peroxidase (HRP), alkaline phosphatase, colloid gold, fluorescein, and a dye.

9. The use of claim 8 wherein the color-developing substrate is selected from the group consisting of 3, 3',5, 5'-tetramethyl benzidine (TMB), 2,2'-azino-bis (3-ethylbenzothiazoline-6-sulfonic acid) (ABTS), and o-phenylenediamine (OPD).

## Patentansprüche

1. Verwendung eines spezifischen Antikörpers gegen das humane Proto-Onkogen HCCR-2 (GenBank Zugriffsnr. AF315598) zur Diagnose von Leberzirrhose *in vitro.*

2. Verwendung nach Anspruch 1, wobei die Diagnose durch Messen der Expressionshöhe des HCCR-2 in einer Probe durch eine Antigen-Antikörper-Bindungsreaktion durchgeführt wird.

3. Verwendung nach Anspruch 2, wobei die Antigen-Antikörper-Bindungsreaktion durch eine Technik, die aus der Gruppe, bestehend aus Enzym-verknüpfter Immunadsorptionstest (ELISA), Radioimmuntest (RIA), Sandwich-Test und Westem-Blot, Immunblot-Analyse oder Immunhistochemie auf einem Polyacrylamidgel, ausgewählt ist, detektiert wird.

4. Verwendung nach Anspruch 1 oder 2, wobei der HCCR-2-spezifische Antikörper ein polyklonaler Antikörper ist.

5. Verwendung nach einem der Ansprüche 2 bis 4, wobei die Probe ein Gewebe, Serum oder Blutplasma ist.

6. Verwendung nach Anspruch 2, wobei der spezifische Antikörper gegen das humane Proto-Onkogen HCCR-2 Teil eines diagnostischen Kits ist, der darüber hinaus eine positive Kontrollgruppe, enthaltend ein HCCR-2-Standardantigen, und eine negative Kontrollgruppe, enthaltend Antiserum eines Tiers, in das kein Antigen injiziert wurde, umfasst.

7. Verwendung nach Anspruch 6, wobei der Kit darüber hinaus
a) ein sekundäres Antikörperkonjugat mit einer konjugierten Markierungssubstanz, die eine Färbung durch Reaktion mit einem Substrat erzeugt;
b) eine Farbentwicklungssubstratlösung, die mit der Markierungssubstanz reagiert, um eine Färbung zu erzeugen;
c) eine Waschlösung, die in jedem Schritt anzuwenden ist; und
d) eine Enzymstoplösung
umfasst.

8. Verwendung nach Anspruch 7, wobei die Markierungssubstanz des sekundären Antikörperkonjugats aus der Gruppe, bestehend aus Meerrettichperoxidase (HRP), alkalischer Phosphatase, kolloidalem Gold, Fluorescein und einem Farbstoff, ausgewählt ist.

9. Verwendung nach Anspruch 8, wobei das Farbentwicklungssubstrat aus der Gruppe, bestehend aus 3,3',5,5'-Tetramethylbenzidin (TMB), 2,2'-Azino-bis (3-ethylbenzothiazolin-6-sulfonsäure (ABTS) und o-Phenylendiamin (OPD), ausgewählt ist.

## Revendications

1. Utilisation d'un anticorps spécifique dirigé contre la protéine prote-oncogène HCCR-2 humaine (No. d'accès GenBank AF315598) pour diagnostiquer une cirrhose hépatique *in vitro.*

2. Utilisation selon la revendication 1, le diagnostic étant réalisé en mesurant le niveau d'expression de la protéine HCCR-2 dans un échantillon prélevé, par une réaction de liaison antigène-anticorps.

3. Utilisation selon la revendication 2, dans laquelle la réaction de liaison antigène-anticorps est détectée par une technique quelconque choisie dans le groupe constitué par un test ELISA (« enzyme-linked immunosorbent assay »), un test radio-immunologique (RIA), un test sandwich et une analyse Western blot, une analyse par immunotransfert ou une immunohistochimie sur un gel de polyacrylamide.

4. Utilisation selon la revendication 2 ou 3, dans laquelle l'anticorps spécifique de HCCR-2 est un anticorps polyclonal.

5. Utilisation selon l'une quelconque des revendications 2 à 4, dans laquelle l'échantillon prélevé est un tissu, du sérum ou du plasma sanguin.

6. Utilisation selon la revendication 2, dans laquelle l'anticorps spécifique dirigé contre la protéine proto-oncogène HCCR-2 humaine fait partie d'un kit de diagnostic comprenant en outre un groupe de contrôle positif contenant un antigène HCCR-2 étalon et un groupe de contrôle négatif contenant de l'antisérum d'un animal dans lequel l'antigène n'est pas injecté.

7. Utilisation selon la revendication 6, dans laquelle le kit comprend en outre
a) un conjugué d'anticorps secondaire ayant une substance de marquage conjuguée produisant une couleur en réagissant avec un substrat ;
b) une solution de substrat développant une couleur en réagissant avec la substance de marquage pour produire une couleur ;
c) une solution de lavage à utiliser dans chaque étape ; et
d) une solution d'arrêt enzymatique.

8. Utilisation selon la revendication 7, dans laquelle la substance de marquage du conjugué d'anticorps secondaire est choisie dans le groupe constitué par la peroxydase de raifort (HRP), la phosphatase alcaline, l'or colloïdal, la fluorescéine et un colorant.

9. Utilisation selon la revendication 8, dans laquelle le substrat développant une couleur est choisi dans le groupe constitué par la 3,3',5,5'-tétraméthyl-benzidine (TMB), le 2,2'-azino-bis(acide 3-éthylbenzothiazoline-6-sulfonique) (ABTS) et l'o-phénylène-diamine (OPD).
